# FASCICULE DE BREVET EUROPEEN

(11) **EP 0 974 375 B1**
(45) Date de publication et mention de la délivrance du brevet: **02.05.2003**
(21) Numéro de dépôt: 99490019.9
(22) Date de dépôt: 13.07.1999
(51) Int. Cl.: A61M 29/02

(54) **Dispositif à ballonnet à diamètre nominal ajustable**
Vorrichung mit einem Ballon mit nominal einstellbarem Durchmesser
Balloon device with nominally adjustable diameter

(30) Priorité: 22.07.1998 FR 9809527
(43) Date de publication de la demande: 26.01.2000
(73) Titulaire: Prodimed, S.A., 60530 Neuilly-en-Thelle (FR)
(72) Inventeur: Camus, Michel, 59175 Templemars (FR)
(74) Mandataire: Ecrepont, Robert

(56) Documents cités:
- EP-A- 0 843 990
- US-A- 5 246 421
- US-A- 5 545 209
- US-A- 5 549 551

## Description

L'invention se rapporte à un dispositif à ballonnet à diamètre nominal ajustable.

L'invention concerne avantageusement les dispositifs à ballonnet pour réaliser, à titre d'exemple, des embolectomies, des thrombectomies, des occlusions, des extractions de calculs biliaires.

L'état de la technique comprend de nombreux documents qui décrivent des dispositifs à ballonnet du type précité.

Par dispositif à ballonnet, on désigne un dispositif qui comprend au moins un conduit tubulaire flexible dont une extrémité dite distale, de diamètre déterminé, destinée à être introduite dans un conduit naturel d'un corps vivant, est équipée d'au moins une poche à paroi tubulaire élastiquement déformable qui, par injection d'un fluide à travers au moins un canal du conduit tubulaire, peut être radialement dilatée autour du conduit tubulaire porteur, et ce, depuis un diamètre externe équivalent à celui de l'extrémité distale, jusqu'à un diamètre notablement supérieur, dit diamètre nominal.

Par diamètre nominal du ballonnet on désigne la dimension qui, mesurée dans un plan orthogonal à l'axe longitudinal du dispositif, représente la valeur maximale à laquelle la poche constitutive de ce ballonnet peut être dilatée sans risque de déchirement de sa paroi.

Ce diamètre nominal du ballonnet est lié à l'élasticité du matériau constitutif de la paroi de la poche et dépend du diamètre qu'elle affecte à l'état non dilaté, de l'épaisseur de sa paroi, de sa dimension longitudinale.

L'épaisseur de la paroi de la poche et son diamètre à l'état non dilaté dépendent, quant à eux, du diamètre externe du conduit tubulaire porteur, lequel est choisi en fonction du diamètre interne du canal naturel dans lequel le dispositif doit être introduit.

Les documents EP 0.843.990, US 5.216.421, US 5.545.209, US 5.549.551 décrivent des cathéters d'angioplastie à ballonnets conventionnels dans lesquels le ballonnet est plissé à l'état non gonflé, et retrouve une forme prédéterminée par gonflement de l'enveloppe du ballonnet réalisé en matériau rigide.

Pour pratiquer des interventions, telles les embolectomies dans des conditions de sécurité optimales pour le patient, les praticiens doivent disposer de dispositifs à ballonnet d'une pluralité de diamètres nominaux, ce qui est onéreux.

Un résultat que l'invention vise à obtenir est un dispositif à ballonnet dont le diamètre nominal du ballonnet peut être ajusté, à différentes valeurs de diamètres nominaux.

En permettant de remplacer plusieurs dispositifs de l'état de la technique de tailles distinctes, le dispositif selon l'invention permet donc de réduire notablement la quantité de dispositifs à ballonnet dont doit disposer un hôpital, une clinique afin de pouvoir faire face aux interventions chirurgicales.

D'autres résultats que l'invention permet d'obtenir seront plus après annoncés.

L'invention a pour objet un dispositif à ballonnet à diamètre nominal ajustable comprenant :
au moins un conduit tubulaire flexible dont une extrémité dite distale, de diamètre déterminé, est équipée d'au moins une poche à paroi tubulaire élastiquement déformable qui, par injection d'un fluide, peut être radialement dilatée et ce, depuis un diamètre externe, jusqu'à un diamètre nominal ajustable,
le dispositif comprenant à son extrémité distale au moins un tronçon de manchon tubulaire qui, de diamètre interne sensiblement égal au diamètre externe du ballonnet à l'état non dilaté, est monté sur ce ballonnet de manière axialement coulissante,
ce dispositif étant caractérisé en ce que le tronçon tubulaire de matériau élastiquement déformable constitutif de ladite poche est élastiquement tendu entre les deux zones de fixation au conduit tubulaire qui le supporte.

L'invention sera bien comprise à la lecture de la description ci-après faite en regard du dessin à titre d'exemple non limitatif, en regard du dessin ci-annexé qui représente :

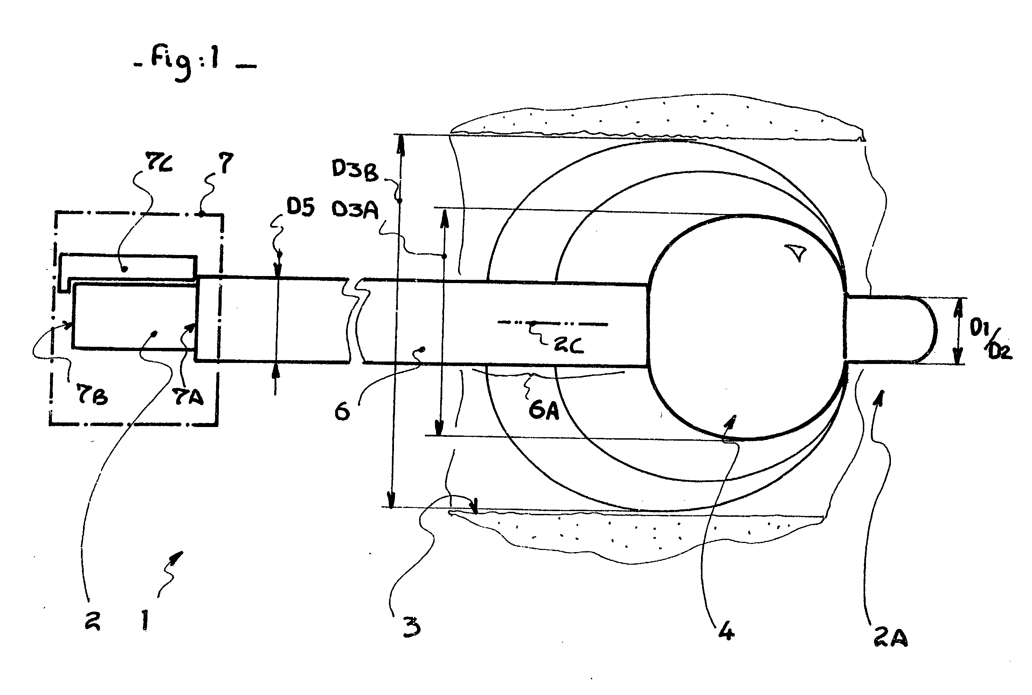

- figure 1 : le dispositif de l'invention vu de profil, avec, en traits forts, le ballonnet représenté selon une première taille nominale et, en traits fin, le même ballonnet selon deux tailles nominales supplémentaires,

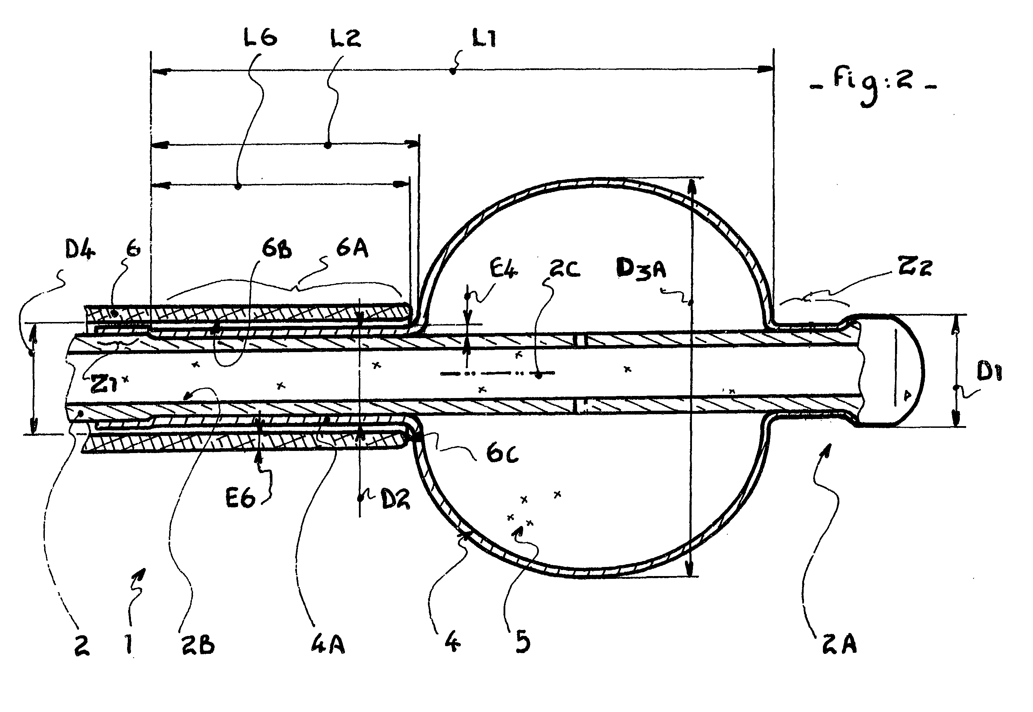

- figure 2 : à plus grande échelle, l'extrémité distale du dispositif de la figure 1.

En se reportant au dessin, on voit un dispositif 1 à ballonnet.

Ce dispositif à ballonnet comprend au moins un conduit tubulaire flexible 2 dont une extrémité dite distale 2A, de diamètre D1 déterminé, destinée à être introduite dans un conduit 3 naturel d'un corps vivant (non représenté), est équipée d'au moins une poche 4 à paroi 4A tubulaire élastiquement déformable qui, par injection d'un fluide 5 à travers au moins un canal 2B du conduit tubulaire 2, peut être radialement dilatée, et ce, depuis un diamètre externe D2 équivalent à celui D1 de l'extrémité distale 2A, jusqu'à un diamètre D3A, D3B notablement supérieur, dit diamètre nominal.

Le diamètre nominal D3A, D3B du ballonnet 4 est la dimension qui, mesurée dans un plan orthogonal à l'axe longitudinal 2C du dispositif 2, représente la valeur maximale à laquelle la poche 4 constitutive de ce ballonnet 4 peut être dilatée sans risque de déchirement de sa paroi 4A.

Ce diamètre nominal D3A, D3B du ballonnet 4 est lié à l'élasticité du matériau constitutif de paroi 4A de la poche 4 et dépend du diamètre D2 qu'elle affecte à l'état non dilaté, de l'épaisseur E4 de sa paroi 4A, de sa dimension longitudinale L1.

Le dispositif selon l'invention comprend à son extrémité distale au moins un tronçon 6A de manchon tubulaire 6 qui, de diamètre interne D4 sensiblement égal au diamètre externe D2 du ballonnet 4 à l'état non dilaté, est monté sur ce ballonnet 4 de manière axialement coulissante.

Selon l'invention :
- le manchon 6 est constitué pour résister à une action radialement développée contre sa face interne 6B par la poche 4, lors de la mise en pression de cette dernière par un fluide 5, et
- le dispositif porte un moyen 7 de réglage de la situation longitudinale du manchon 6 à son extrémité distale, de manière telle que la dimension longitudinale L2 d'au moins une fraction de poche 4 entourée par ce manchon 6 puisse être ajustée pour, sur cette fraction déterminée, entraver la dilatation radiale de la paroi 4A de cette poche 4 sous l'effet du fluide sous pression 5.

Le manchon 6 a donc une fonction de rétention radiale de la poche 4.

Par cela, le dispositif à ballonnet selon l'invention est remarquable en ce que le diamètre nominal de son ballonnet 4 est ajustable entre deux valeurs extrêmes D3A et D3B, dont une valeur minimale D3A et une valeur maximale D3B.

Le moyen 7 de réglage de la situation longitudinale dudit manchon 6 à l'extrémité distale du dispositif est de type réglable par fraction déterminée de la dimension longitudinale L1 de la poche 4.

Par cela le diamètre nominal du ballonnet peut être ajusté par fraction déterminée de diamètre.

Par exemple, tel que cela a été annoncé précédemment, le diamètre du ballonnet peut être ajusté de manière telle que sa taille puisse être calibrée à différentes valeurs classiques telles que celles de dispositifs de tailles dites "CHARRIERE" trois, quatre et cinq.

Un dispositif selon l'invention peut donc remplacer au moins trois dispositifs de tailles distinctes de l'état de la technique.

Par exemple, un prototype réalisé par les soins de l'inventeur permet de vérifier qu'avec une paroi tubulaire élastique 4 dont le diamètre transversal D2 à l'état non dilaté est d'un millimètre virgule deux (1,2 mm), le dispositif de l'invention permet de constituer des ballonnets de diamètres nominaux D3A à D3B de cinq, puis sept, puis neuf millimètres (5, puis, 7, puis 9 mm) c'est à dire des ballonnets de taille correspondant à ceux qui pourraient être obtenus avec des dispositifs de tailles dites de CHARRIERE trois, quatre et cinq.

Le manchon tubulaire 4 utilisé pour ce prototype est d'un diamètre externe D2 d'un millimètre et demi (1,5 mm).

On peut noter que :
- pour offrir un diamètre nominal D3B de neuf millimètres (9 mm), le diamètre transversal de la paroi tubulaire élastique d'un dispositif de l'état de la technique à l'état non dilaté est classiquement sensiblement supérieur à un millimètre et soixante six centièmes de millimètre (1,66 mm), soit une taille dite CHARRIERE 5,
- pour offrir un diamètre nominal D3B de cinq millimètres (5 mm), le diamètre transversal de la paroi tubulaire élastique d'un dispositif de l'état de la technique à l'état non dilaté est classiquement sensiblement supérieur à un millimètre (1 mm), soit une taille de CHARRIERE 3.

Si on considère ces dimensions à titre d'exemple, on peut noter qu'avec un encombrement diamétral D5 inférieur à un dispositif de l'état de la technique de taille CHARRIERE 5, le dispositif de l'invention permet de disposer d'un ballonnet de tailles équivalents à ceux de dispositifs de tailles dites CHARRIERE 3, 4 et 5.

Egalement, l'invention permet, avec un unique dispositif, de disposer de ballonnets de plusieurs tailles dont l'étendue de la face externe en contact avec la face interne du conduit vasculaire est relativement limitée par rapport à ce qui pourrait être obtenu avec un dispositif de l'état de la technique.

Le fait que l'étendue de la surface du ballonnet en contact avec la surface interne du conduit vasculaire soit limitée permet, notamment pour les diamètres intermédiaires entre le diamètre à l'état non dilaté et le diamètre maximal, de disposer d'un ballonnet ferme et dont la paroi s'efface difficilement sous l'effet d'un thrombus, lors de la traction du ballonnet dans le conduit vasculaire.

Tel que cela est annoncé plus avant, un tel résultat ne pourrait être obtenu avec un ballonnet de l'état de la technique, d'une part, de diamètre nominal supérieur à celui nécessaire pour l'intervention et, d'autre part, dont les diamètres intermédiaires entre le diamètre nominal et le diamètre à l'état non dilaté, seraient obtenus uniquement par ajustement de la quantité de fluide sous pression.

En effet, la dilatation d'un tel ballonnet à un diamètre inférieur au diamètre nominal induit une mollesse certaine de sa paroi et une efficacité douteuse dans le rôle de décollement puis déplacement de thrombus qui est affecté au dispositif.

En d'autres termes, l'utilisation d'un tel ballonnet de l'état de la technique permettrait l'effacement local de sa paroi et le franchissement d'un thrombus qu'il aurait dû détacher de la paroi vasculaire.

De manière remarquable :
- le moyen 7 de réglage de la situation longitudinale dudit manchon 6 à l'extrémité distale du dispositif détermine deux positions limites dont :
   . une position qui établit le plus petit diamètre nominal pouvant être obtenu avec le ballonnet du dispositif, et
   . une autre position qui, quant à elle, établit le plus grand diamètre nominal de ce ballonnet,
- la position limite dans laquelle le plus petit diamètre nominal peut être obtenu est une position dans laquelle le manchon 6 se trouve partiellement engagé sur la paroi tubulaire élastique 4 de manière telle que, y compris à l'état non dilaté de cette paroi 4, ledit manchon 6 ne recouvre que la fraction longitudinale de paroi à contraindre pour constituer le ballonnet de plus petit diamètre nominal.

De préférence, la position limite dans laquelle le plus grand diamètre nominal peut être obtenu est une position dans laquelle le manchon 6 se trouve totalement dégagé de la paroi tubulaire élastique 4.

Le moyen 7 de réglage de la situation longitudinale dudit manchon 6 à l'extrémité distale du dispositif est de type actionnable au moins depuis l'extrémité proximale du dispositif, c'est à dire au moins depuis l'extrémité du dispositif opposée à celle distale qui porte la poche 4.

Dans une forme préférée de réalisation, le moyen 7 de réglage de la situation longitudinale dudit manchon 6 à l'extrémité distale du dispositif comprend :
- un tube flexible 6 qui, monté coulissant autour du conduit tubulaire 2 porteur de la poche 4, présente :
   . d'une part, une extrémité distale constituant le manchon et,
   . d'autre part, une extrémité proximale quant à elle située au niveau de l'extrémité du dit conduit tubulaire porteur 2,
- aux moins deux butées 7A, 7B chacune au moins indirectement portée par l'une des extrémités proximales du tube flexible et du conduit tubulaire,
- un organe 7C de réglage de l'écartement de ces butées 7A, 7B.

Dans une forme de réalisation, le matériau constitutif du manchon 6, l'épaisseur E6 et la dimension longitudinale L6 de la paroi de ce manchon 6 sont choisis de manière telle que ledit manchon 6 assure sa fonction de contention d'au moins une fraction de la dimension longitudinale L de la poche 4 en s'opposant à la dilatation de cette partie sous l'effet du fluide 5 sous pression qu'elle reçoit.

De même, le matériau constitutif de la poche 4, l'épaisseur E4 et la dimension longitudinale L1 de la paroi 4A de cette poche 4 sont choisis de manière telle que ladite poche 4 assure sa fonction sous l'effet du fluide 5 sous pression qu'elle reçoit.

Classiquement, la poche 4 est constituée au moyen d'un tronçon tubulaire de matériau élastiquement déformable qui, emboîté sur un conduit tubulaire 2, a ses deux extrémités qui sont chacune étroitement associées au dit conduit 2, par exemple par ligature dans deux zones périphériques Z1, Z2.

Pour, de manière drastique, limiter le risque de plissement de la paroi élastiquement déformable 4A de la poche 4, lors du déplacement du manchon 6, le tronçon tubulaire de matériau élastiquement déformable constitutif de ladite poche 4 est élastiquement tendu entre les deux zones Z1, Z2 de fixation au conduit tubulaire 2 qui le supporte.

Une mise en tension dudit tronçon tubulaire est donc réalisée préalablement à sa fixation, notamment par ligature, au conduit tubulaire support dans les zones périphériques Z1, Z2.

De manière remarquable, le manchon tubulaire de rétention présente, à son extrémité libre, une surface terminale 6C qui se raccorde à la face interne 6B du tronçon par un congé de manière telle que l'application énergique de la paroi souple 4 contre ladite surface terminale 6C n'induise pas de dommage à la paroi 4, lors de son application par l'effet du fluide sous pression 5.

## Revendications

1. Dispositif (1) à ballonnet comprenant au moins un conduit tubulaire flexible (2) dont une extrémité dite distale (2A), de diamètre (D1) déterminé, est équipée d'au moins une poche (4) à paroi (4A) tubulaire élastiquement déformable qui, par injection d'un fluide (5), peut être radialement dilatée et ce, depuis un diamètre externe (D2), jusqu'à un diamètre nominal ajustable (D3A, D3B),
le dispositif comprenant à son extrémité distale au moins un tronçon (6A) de manchon tubulaire (6) qui, de diamètre interne (D4) sensiblement égal au diamètre externe (D2) du ballonnet (4) à l'état non dilaté, est monté sur ce ballonnet (4) de manière axialement coulissante,
ce dispositif étant **caractérisé en ce que** le tronçon tubulaire (4A) de matériau élastiquement déformable constitutif de ladite poche (4) est élastiquement tendu entre les deux zones (Z1, Z2) de fixation au conduit tubulaire (2) qui le supporte).

2. Dispositif selon la revendication 1 **caractérisé en ce qu'**il comprend un moyen (7) de réglage de la situation longitudinale dudit manchon (6) à l'extrémité distale du dispositif, ce moyen (7) étant de type réglable par fraction déterminée de la dimension longitudinale (L1) de la poche (4).

3. Dispositif selon la revendication 2 **caractérisé en ce que** :
- le moyen (7) de réglage de la situation longitudinale dudit manchon (6) à l'extrémité distale du dispositif détermine deux positions limites dont,
. une position qui établit le plus petit diamètre nominal pouvant être obtenu avec le ballonnet du dispositif, et
. une autre position qui, quant à elle, établit le plus grand diamètre nominal de ce ballonnet,
- la position limite dans laquelle le plus petit diamètre nominal peut être obtenu est une position dans laquelle le manchon (6) se trouve partiellement engagé sur la paroi tubulaire élastique (4) de manière telle que, y compris à l'état non dilaté de cette paroi (4), ledit manchon (6) ne recouvre que la fraction longitudinale de paroi à contraindre pour constituer le ballonnet de plus petit diamètre nominal.

4. Dispositif selon la revendication 2 ou 3 **caractérisé en ce que** le moyen (7) de réglage de la situation longitudinale dudit manchon (6) à l'extrémité distale du dispositif est de type actionnable au moins depuis l'extrémité proximale du dispositif, c'est à dire au moins depuis l'extrémité du dispositif opposée à celle distale qui porte la poche (4).

5. Dispositif selon l'une quelconque des revendications 1 à 4 **caractérisé en ce que** le moyen (7) de réglage de la situation longitudinale dudit manchon (6) à l'extrémité distale du dispositif comprend :
- un tube flexible (6) qui, monté coulissant autour du conduit tubulaire (2) porteur de la poche (4), présente :
. d'une part, une extrémité distale constituant le manchon et,
. d'autre part, une extrémité proximale quant à elle située au niveau de l'extrémité du dit conduit tubulaire porteur (2),
- au moins deux butées (7A, 7B) chacune au moins indirectement portée par l'une des extrémités proximales du tube (6) et du conduit tubulaire porteur (2),
- un organe (7C) de réglage de l'écartement de ces butées (7A, 7B).

6. Dispositif selon l'une quelconque des revendications 1 à 5 **caractérisé en ce que** le manchon tubulaire de rétention présente, à son extrémité libre, une surface terminale (6C) qui se raccorde à la face interne (6B) du tronçon par un congé de manière telle que l'application énergique de la paroi souple (4) contre ladite surface terminale (6C) n'induise pas de dommage à la paroi (4).

## Claims

1. Balloon device (1) comprising at least one flexible tube (2), of which one end, termed distal (2A), of defined diameter (D1), is equipped with at least one pocket (4) having an elastically deformable tubular wall (4A) which can be radially dilated, by the injection of a fluid (5), from an outside diameter (D2) to an adjustable nominal diameter (D3A, D3B),
said device comprising at its distal end at least one section (6A) of tubular sleeve (6), the inside diameter (D4) of which is substantially equal to the outside diameter (D2) of the balloon (4) in the non-dilated state and which is mounted on said balloon (4) so as to slide axially,
said device being **characterised in that** the tubular portion (4A) of elastically deformable material constituting said pocket (4) is stretched elastically between the two regions (Z1, Z2) for fixing it to the tube (2) which supports it.

2. Device according to claim 1, **characterised in that** it comprises means (7) for adjusting the longitudinal position of said sleeve (6) at the distal end of the device, these means (7) being of the type which can be adjusted by a defined fraction of the longitudinal dimension (L1) of the pocket (4).

3. Device according to claim 2, **characterised in that**:
- the means (7) for adjusting the longitudinal position of said sleeve (6) at the distal end of the device determines two limit positions, which are
. a position which establishes the smallest nominal diameter which can be obtained with the balloon of the device, and
. another position which, for its part, establishes the largest nominal diameter of this balloon,
- the limit position in which the smallest nominal diameter can be obtained is a position in which the sleeve (6) is partially engaged on the elastic tubular wall (4) in such a way that, even in the non-dilated state of this wall (4), said sleeve (6) only covers the longitudinal wall fraction to be constrained to constitute the balloon having the smallest nominal diameter.

4. Device according to claim 2 or 3, **characterised in that** the means (7) for adjusting the longitudinal position of said sleeve (6) at the distal end of the device is of a type which can be actuated at least from the proximal end of the device, i.e. at least from the end of the device opposite the distal end which carries the pocket (4).

5. Device according to any one of claims 1 to 4, **characterised in that** the means (7) for adjusting the longitudinal position of said sleeve (6) at the distal end of the device comprise:
- a flexible tube (6) which is mounted sliding around the tube (2) carrying the pocket (4) and has:
. firstly, a distal end constituting the sleeve and,
. secondly, a proximal end which is situated at the level of the end of said carrying tube (2),
- at least two stops (7A, 7B), each at least indirectly carried by one of the proximal ends of the sleeve tube (6) and of the carrying tube (2),
- a member (7C) for adjusting the spacing of these stops (7A, 7B).

6. Device according to any one of claims 1 to 5, **characterised in that** the tubular retention sleeve has, at its free end, an end surface (6C) which is connected to the internal face (6B) of the sleeve section by a fillet in such a way that the application of energy from the flexible wall (4) against said end surface (6C) does not cause any damage to the wall (4).

## Patentansprüche

1. Vorrichtung (1) mit einem Ballon, die mindestens eine flexible Rohrleitung (2) mit einem vorgegebenen Durchmesser (D1) aufweist, die an ihrem distalen Ende (2A) mit mindestens einem Beutel (4) mit einer elastisch verformbaren schlauchförmigen Wand (4A) ausgestattet ist, die durch Injektion eines Fluids (5) radial dilatiert werden kann von einem äußeren Durchmesser (D2) bis zu einem einstellbaren nominellen Durchmesser (D3A, D3B),
wobei die Vorrichtung an ihrem distalen Ende mindestens einen Abschnitt (6A) einer Schlauchmanschette (6) mit einem Innendurchmesser (D4), der im Wesentlichen gleich dem Außendurchmesser (D2) des Ballons (4) im nichtdilatierten Zustand ist, aufweist, die auf diesem Ballon (4) axial verschiebbar befestigt ist,
wobei diese Vorrichtung **dadurch gekennzeichnet ist, dass** der schlauchförmige Abschnitt (4A) aus einem elastisch verformbaren Material, das den Beutel (4) aufbaut, zwischen den beiden Fixierungszonen (Z1, Z2) bis zu der Rohrleitung (2) die ihn trägt, elastisch gespannt ist.

2. Vorrichtung nach Anspruch 1, **dadurch gekennzeichnet, dass** sie eine Einrichtung (7) zur Einstellung der longitudinalen Position der Manschette (6) am distalen Ende der Vorrichtung aufweist, wobei diese Einrichtung (7) eine solche eines Typs ist, der durch einen vorgegebenen Bruchteil der Längsdimension (L1) des Beutels (4) einstellbar ist.

3. Vorrichtung nach Anspruch 2, **dadurch gekennzeichnet, dass**
- die Einrichtung (7) zur Einstellung der longitudinalen Position der Manschette (6) am distalen Ende der Vorrichtung zwei Grenzpositionen festlegt, nämlich
• eine Position, die den kleinsten nominellen Durchmesser festlegt, der mit dem Ballon der Vorrichtung erhalten werden kann, und
• eine andere Position, die den größten nominellen Durchmesser dieses Ballons festlegt,
- wobei die Grenzposition, in der der kleinste nominelle Durchmesser erhalten werden kann, eine Position ist, in der die Manschette (6) teilweise so auf die elastische rohrförmige Wand (4) aufgeschoben ist, dass im nichtdilatierten Zustand dieser Wand (4) die Manschette (6) nur den longitudinalen Teil der Wand bedeckt, der zusammenzudrücken ist, um den Ballon mit dem kleinsten nominallen Durchmesser zu erzeugen.

4. Vorrichtung nach Anspruch 2 oder 3, **dadurch gekennzeichnet, dass** die Einrichtung (7) zur Einstellung der longitudinalen Position der Manschette (6) am distalen Ende der Vorrichtung von einem solchen Typ ist, der mindestens ab dem proximalen Ende der Vorrichtung betätigbar ist, d.h. mindestens ab dem Ende der Vorrichtung, das dem distalen Ende, das den Beutel (4) trägt, gegenüberliegt.

5. Vorrichtung nach einem der Ansprüche 1 bis 4, **dadurch gekennzeichnet, dass** die Einrichtung (7) zur Einstellung der longitudinalen Position der Manschette (6) an dem distalen Ende der Vorrichtung umfasst:
- einen Schlauch (6), der um die Rohrleitung (2), herum, die den Beutel (4) trägt, verschiebbar befestigt ist und aufweist:
• einerseits ein distales Ende, welches die Manschette darstellt, und
• andererseits ein proximales Ende, das seinerseits im Bereich des Endes der Träger-Rohrleitung (2) angeordnet ist,
- mindestens zwei Anschläge (7A, 7B), die jeweils mindestens indirekt von einem der proximalen Enden des Schlauches (6) und der Träger-Rohrleitung (2) getragen werden,
- ein Organ (7C) zur Einstellung des Abstandes zwischen diesen Anschlägen (7A, 7B).

6. Vorrichtung nach einem der Ansprüche 1 bis 5, **dadurch gekennzeichnet, dass** die Retentions-Schlauchmanschette an ihrem freien Ende eine Stirnfläche (6C) aufweis, die durch eine Hohlkehle mit der inneren Oberfläche (6B) des Abschnittes so in Verbindung steht, dass ein energisches Andrücken der weichen Wand (4) gegen die Stirnfläche (6C) keine Beschädigung der Wand (4) hervorruft.
